(19) Europäisches Patentamt
European Patent Office
Office européen des brevets

(11) **EP 4 760 647 A1**

(12) **EUROPEAN PATENT APPLICATION**
published in accordance with Art. 153(4) EPC

(43) Date of publication:
**17.06.2026 Bulletin 2026/25**

(51) International Patent Classification (IPC):
*G06V 10/25* (2022.01)

(21) Application number: **24871043.6**

(22) Date of filing: **27.09.2024**

(86) International application number:
**PCT/CN2024/122024**

(87) International publication number:
**WO 2025/067508 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.09.2023 CN 202311269305**

(71) Applicant: **Shanghai United Imaging Healthcare
Co., Ltd.
Shanghai 201807 (CN)**

(72) Inventors:
• **SHI, Yiran**
 **Shanghai 201807 (CN)**
• **CAO, Wenjing**
 **Shanghai 201807 (CN)**
• **ZHAO, Liyi**
 **Shanghai 201807 (CN)**

(74) Representative: **Murgitroyd & Company
165-169 Scotland Street
Glasgow G5 8PL (GB)**

(54) **METHOD AND APPARATUS FOR PROCESSING SCANNING DATA, AND COMPUTER DEVICE AND STORAGE MEDIUM**

(57) Disclosed is a method for processing scanning data. The method comprises: acquiring scanning data (S210); and performing joint processing on the scanning data by using a preset deep learning network, so as to obtain a processing result of the scanning data (S220), wherein the scanning data comprises scanning data of a plurality of different energy spectrums, and the deep learning network is configured to perform feature extraction on the scanning data of each energy spectrum among the scanning data of the plurality of different energy spectrums, and perform feature extraction on the scanning data of all energy spectrums among the scanning data of the plurality of different energy spectrums. Further disclosed are an apparatus for processing scanning data, and a computer device and a storage medium.

```
                                    ⌐ S210
┌─────────────────────────────────────┐
│                                     │
│        Acquire scanning data         │
│                                     │
└─────────────────────────────────────┘
                   │
                   ▼            ⌐ S220
┌─────────────────────────────────────┐
│                                     │
│ Perform processing on the scanning  │
│ data by using a predetermined deep  │
│ learning network, to obtain a       │
│ processing result of the scanning   │
│ data                                 │
└─────────────────────────────────────┘
```

FIG. 2

**Description**

**CROSS-REFERENCE TO RELATED APPLICATIONS**

The present disclosure claims priority to Chinese patent application No. 202311269305.8 filed with the China National Intellectual Property Administration on September 27, 2023, the entire content of which is incorporated by reference herein for all purposes.

**TECHNICAL FIELD**

The present disclosure relates to the field of medical imaging technologies, and in particular, to a method and device for processing scanning data, and a storage medium.

**BACKGROUND**

For scanning data obtained by computed tomography (CT) and preprocessed data obtained by preprocessing the scanning data, predetermined deep learning networks can be adopted to perform processing such as denoising, region segmentation, and scan result analysis to obtain corresponding image processing results. For multi-spectral scanning data or other different types of scanning data with consistent image structures, in conventional technologies, deep learning networks are usually used to separately process scanning data of each energy spectrum to obtain processing results for the scanning data of each energy spectrum, respectively. In this way, the denoising degree, segmentation accuracy, efficiency, etc., of the processing results are relatively low.

Conventional techniques face an issue in which deep learning networks are used to individually process multi-spectral scanning data, such as various types of scanning data that have consistent image structures. This individual processing approach leads to low accuracy and efficiency. Currently, no effective solution has been proposed to address this problem.

**SUMMARY**

In this embodiment, a method and device for processing scanning data, and a storage medium are provided to solve the problem in the related art that the accuracy and efficiency of processing results are relatively low when various types of scanning data are processed separately.

In a first aspect of the present disclosure, a method for processing scanning data is provided, including acquiring scanning data, and performing joint processing on the scanning data by using a predetermined deep learning network, to obtain a processing result of the scanning data. The scanning data includes scanning data of a plurality of different energy spectra. The deep learning network is configured to extract features from scanning data of each energy spectrum among the scanning data of the plurality of different energy spectra, and extract features from scanning data of all energy spectra among the scanning data of the plurality of different energy spectra.

In the above first aspect, the deep learning network includes both a single extraction layer for extracting features from the scanning data of each energy spectrum and a common extraction layer for extracting features from the scanning data of all energy spectra.

In the above first aspect, features extracted from the scanning data of each energy spectrum are used only by the scanning data corresponding to energy spectrum, and features extracted from the scanning data of all energy spectra are shared by the scanning data of the plurality of different energy spectra.

In the above first aspect, the scanning data are obtained by a medical scanning device. The medical scanning device includes at least one of a photon-counting computed tomography device, an energy spectral computed tomography device, a direct digital radiography device, a single-photon emission computed tomography device, or a positron emission tomography (PET-CT) device.

In the above first aspect, acquiring the scanning data includes: acquiring initial scanning data obtained by photon-counting computed tomography, and dividing the initial scanning data into a plurality of different energy spectral segments to acquire the scanning data of the plurality of different energy spectra corresponding to the plurality of different energy spectral segments respectively.

In the above first aspect, acquiring the scanning data includes acquiring the scanning data of the plurality of different energy spectra corresponding to a plurality of energy spectra respectively based on energy spectral computed tomography.

In the above first aspect, acquiring the scanning data of the plurality of different energy spectra corresponding to the plurality of energy spectra respectively based on energy spectral computed tomography includes: performing computed tomography using at least two different energies respectively to obtain scanning data corresponding to different energy spectra respectively, or performing computed tomography based on two different light sources respectively to obtain scanning data corresponding to different energy spectra respectively.

In the above first aspect, before performing processing on the scanning data by using the predetermined deep learning network, to obtain the processing result of the scanning data, the method further includes using the scanning data as inputs to the deep learning network respectively.

In the above first aspect, performing processing on the scanning data by using the predetermined deep learning network, to obtain the processing result of the scanning data includes: separately extracting data features of the scanning data of each energy spectrum by using the single extraction layer of the deep learning network, to obtain single data features corresponding to the scanning data of the plurality of different energy spectra respectively, jointly extracting data features of the scanning data of the plurality of different energy spectra by using the common extraction layer of the deep learning network, to obtain common data features, and obtaining processing results of the scanning data of the plurality of different energy spectra based on the single data features and the common data features.

In the above first aspect, performing joint processing on the scanning data by using the predetermined deep learning network, to obtain the processing result of the scanning data further includes: performing separate optimization processing on scanning data of each energy spectrum by using the deep learning network based on the single data features, and performing joint optimization processing on the scanning data of the plurality of different energy spectra by using the deep learning network based on the common data features.

In the above first aspect, the method further includes: on a condition that the scanning data are multi-spectral data that have not undergone material decomposition, performing material decomposition on output result of the deep learning network, and constraining a process of the material decomposition based on a predetermined loss function to obtain a material map corresponding to the multi-spectral data, or on a condition that the scanning data are material decomposition data, converting the output result of the deep learning network into target energy spectral data, and calculating a loss function of the target energy spectral data using a loss function structure in the deep learning network.

In the above first aspect, the processing performed by the deep learning network on the scanning data includes at least one of the following processes: denoising processing on the scanning data of the plurality of different energy spectra, extraction of a region of interest from the scanning data of the plurality of different energy spectra, or scan result analysis on the scanning data of the plurality of different energy spectra.

In a second aspect of the present disclosure, a device for processing scanning data is provided, the apparatus including an acquisition module and an output module. The acquisition module is configured to acquire scanning data. The output module is configured to perform processing on the scanning data by using a predetermined deep learning network, to obtain a processing result of the scanning data. The scanning data includes scanning data of a plurality of different energy spectra. The deep learning network is configured to extract features from scanning data of each energy spectrum among the scanning data of the plurality of different energy spectra, and extract features from scanning data of all energy spectra among the scanning data of the plurality of different energy spectra.

In the above second aspect, the deep learning network includes both a single extraction layer for extracting features from the scanning data of each energy spectrum and a common extraction layer for extracting features from the scanning data of all energy spectra.

In the above second aspect, features extracted from the scanning data of each energy spectrum are used only by the scanning data corresponding to energy spectrum, and features extracted from the scanning data of all energy spectra are shared by the scanning data of the plurality of different energy spectra.

In the above second aspect, the acquisition module is further configured to: acquire initial scanning data obtained by photon-counting computed tomography, and divide the initial scanning data into a plurality of different energy spectral segments to acquire the scanning data of the plurality of different energy spectra corresponding to the plurality of different energy spectral segments respectively.

In the above second aspect, the acquisition module is further configured to: acquire the scanning data of the plurality of different energy spectra corresponding to a plurality of energy spectra respectively based on energy spectral computed tomography.

In the above second aspect, the acquisition module is further configured to: perform computed tomography using at least two different energies respectively to obtain scanning data corresponding to different energy spectra respectively, or perform computed tomography based on two different light sources respectively to obtain scanning data corresponding to different energy spectra respectively.

In the above second aspect, the acquisition module is further configured to use the scanning data of the plurality of different energy spectra as inputs to the deep learning network respectively.

In the above second aspect, the output module is further configured to: separately extract data features of the scanning data of each energy spectrum by using the single extraction layer of the deep learning network, to obtain single data features corresponding to the scanning data of the plurality of different energy spectra respectively, jointly extract data features of different types of scanning data by using the common extraction layer of the deep learning network, to obtain common data features, and obtain processing results of the scanning data of the plurality of different energy spectra based on the single data features and the common data features.

In the above second aspect, the output module is further configured to: perform separate optimization processing on the scanning data of each energy spectrum by using the deep learning network based on the single data features, and perform joint optimization processing on the scanning data of the plurality of different energy spectra by using the deep learning network based on the common data features.

In the above second aspect, the output module is further configured to: on a condition that the scanning data are multi-spectral data that have not undergone material decomposition, perform material decomposition on output result of the deep learning network, and constrain a material decomposition process based on a predetermined loss function to obtain a material map corresponding to the multi-spectral data, or on a condition that the scanning data are material decomposition data, convert the output result of the deep learning network into target energy spectral data, and calculate a loss function of the target energy spectral data using a loss function structure in the deep learning network.

In the above second aspect, the output module is further configured to perform at least one of the following processes: denoising processing on the scanning data of a plurality of different energy spectra, extraction of a region of interest from the scanning data of a plurality of different energy spectra, or scan result analysis on the scanning data of a plurality of different energy spectra.

In a third aspect of the present disclosure, a computer apparatus is provided, which includes a processor and a memory. The memory stores a computer program executable by the processor. The processor, when executing the computer program, implements the method for processing scanning data according to any one of the above first aspect.

In a fourth aspect of the present disclosure, a computer-readable storage medium is provided, which has a computer program stored thereon. The computer program, when executed by a processor, causes the processor to implement the method for processing scanning data according to any one of the above first aspect.

## BRIEF DESCRIPTION OF THE DRAWINGS

The above and other aspects, features and advantages of the present disclosure will become apparent and easily understood from the description of the embodiments with reference to the following accompany drawings:

FIG. 1 is a block diagram illustrating a configuration of a terminal for implementing a method for processing scanning data according to an embodiment of the present disclosure;

FIG. 2 is a flow diagram of a method for processing scanning data according to an embodiment of the present disclosure;

FIG. 3 is a flow diagram of a method for processing scanning data according to another embodiment of the present disclosure;

FIG. 4 is a flow diagram of a method for denoising multi-spectral scanning data according to an embodiment of the present disclosure;

FIG. 5 is a flow diagram of a method for image segmentation of multi-spectral scanning data according to an embodiment of the present disclosure; and

FIG. 6 is a block diagram illustrating a configuration of a device for processing scanning data according to an embodiment of the present disclosure.

## DETAILED DESCRIPTION OF THE EMBODIMENTS

To make the objectives, features and advantages of the present disclosure more apparent and understandable, the technical solutions in the embodiments of the present disclosure will be clearly and completely described below with reference to the accompanying drawings. Apparently, the described embodiments are part of the embodiments of the present disclosure, rather than all of them. Based on the embodiments in the present disclosure, all other embodiments obtained by those of ordinary skill in the art without making creative efforts fall within the protection scope of the present disclosure.

In this document, descriptions referring to the terms "one embodiment", "some embodiments", "examples", "specific examples", or "some examples" etc., mean that a specific feature, structure, material, or characteristic described in connection with the embodiment or example is included in at least one embodiment or example of the present disclosure. In the present disclosure, the schematic representations of the above terms do not necessarily refer to the same embodiment or example. Furthermore, the specific features, structures, materials, or characteristics described may be combined in any suitable manner in any one or more embodiments or examples.

Although the terms "first" and "second" may be used herein to describe various features or elements, these features or elements should not be limited by these terms unless otherwise specified. These terms may be used to distinguish one feature or element from another. Therefore, a first feature or element described below may be referred to as a second feature or element, and similarly, a second feature or element described below may be referred to as a first feature or element without departing from the scope of the present disclosure. In the description of the present disclosure, "a plurality of" means at least two, such as two, three, etc., unless otherwise clearly and specifically defined.

The terms used herein are for the objectives of describing specific embodiments only and are not intended to limit the present disclosure. For example, as used herein, the singular form "a" is also intended to include the plural form, unless the context clearly indicates otherwise. It will be further understood that when used herein, the term "includes" specifies the presence of stated features, steps, operations, elements, and/or components, but does not exclude the presence or addition of one or more other features, steps, operations, elements, components, and/or groups thereof. As used herein, the term "and/or" includes any and all combinations of one or more of the associated listed items, and may be abbreviated as "/".

It should be understood that when a feature or element is referred to as being "connected", "attached" or "coupled" to another feature or element, it may be directly connected, attached or coupled to the other feature or element, or intervening features or elements may be present. In contrast, when a feature or element is referred to as being "directly connected", "directly attached" or "directly coupled" to another feature or element, there are no intervening features or elements present. Although described or illustrated with respect to one embodiment, the features and elements so described or illustrated may be applied to other embodiments.

In an aspect of the present disclosure, a method for processing scanning data is provided. The method may be implemented in a computer apparatus such as a terminal or a server. The terminal includes a fixed terminal such as a desktop computer, and a mobile terminal such as a notebook computer, a tablet computer, a mobile phone, and the like. Details of the embodiments of the present disclosure will be described by taking the execution of the method for processing scanning data provided by the present disclosure on a terminal as an example.

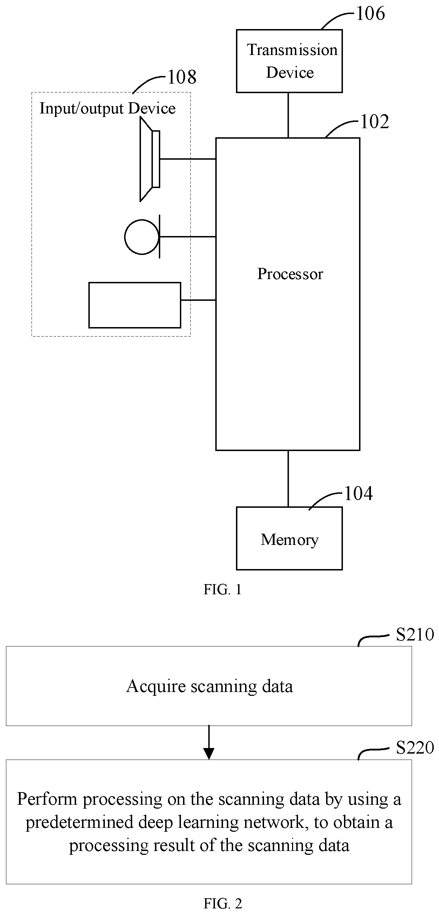

FIG. 1 is a block diagram illustrating a configuration of a terminal for implementing the method for processing scanning data according to an embodiment of the present disclosure. As shown in FIG. 1, the terminal may include one or more (only one is shown in FIG. 1) processors 102 and a memory 104 for storing data. The processor 102 may include, but is not limited to, a processing device such as a central processing unit (CPU), a microcontroller unit (MCU), a digital signal processor (DSP), and an embedded processor. The processor may be realized by an application-specific integrated circuit, a programmable logic device, a system on chip, etc. The terminal may further include a transmission device 106 for communication functions and an input/output device 108. Those of ordinary skill in the art can understand that the configuration shown in FIG. 1 is only schematic and does not limit the configuration of the terminal. For example, the terminal may include more or fewer components than those shown in FIG. 1, or have a different configuration from that shown in FIG. 1.

The memory 104 may be used to store computer programs, such as software programs and modules of application software, such as the computer program corresponding to the method for processing scanning data in this embodiment. The processor 102 executes various functional applications and data processing by running the computer program stored in the memory 104. The memory 104 may include a high-speed random access memory, and may also include a non-transitory memory, such as one or more magnetic storage devices, flash memory, or other non-transitory solid-state memories. In some examples, the memory 104 may further include memories remotely located relative to the processor 102, and these remote memories may be connected to the terminal through a wired or wireless network. Examples of wireless networks include, but are not limited to, the Internet, the Internet of Things, mobile communication networks, and combinations thereof.

The transmission device 106 is configured to receive data from the outside or send data to the outside via network communication. Network communication includes wireless network communication provided by a communication service provider of the terminal. In an example, the transmission device 106 includes a network interface controller (NIC), which can be connected to other network devices through a base station to transmit information via the Internet. In an example, the transmission device 106 may be a radio frequency (RF) module configured to wireless access to the Internet.

In an embodiment of the present disclosure, a method for processing scanning data is provided. As shown in FIG. 2, the method includes steps S210 and S220.

In step S210, scanning data are acquired. The scanning data are obtained by a medical scanning device. The medical scanning device includes at least one of a photon-counting computed tomography (PCD-CT) device, an energy spectral computed tomography (spectral CT) device, a direct digital radiography (DR) device, a single-photon emission computed tomography (SPECT) device, a positron emission tomography (PET-CT) device or other medical scanning devices.

In some embodiments, the scanning data includes scanning data with consistent image structures but differences in other specific aspects, i.e., different types of scanning data with consistent image structures. These different types of scanning data may include different types of scanning data obtained by scanning a same scanning object (for example, a body part at a same physical position) using different scanning sequences, and these scanning data can represent image structures corresponding to structures of the scanning object. Since the same scanning object is scanned, the image structures represented by these scanning data are consistent. These different types of scanning data may also include preprocessed data obtained by preprocessing collected scanning data, such as material decomposition data obtained by performing material decomposition on the scanning data. From the image perspective, the scanning data with consistent image structures includes at least two types of images whose structural compositions correspond to each other. From scanning and reconstruction perspective, the scanning data with consistent image structures include at least two types of scanning data obtained under the condition of consistent scanning range, reconstruction range, and reconstruction parameters. For example, for different types of scanning data, on a condition that in the images, the pixel positions corresponding to the heart are consistent and the pixel positions corresponding to artifacts are consistent across these scanning data, then these scanning data are scanning data with consistent image structures.

The above different types of scanning data may be scanning data of a plurality of different energy spectra obtained by scanning a scanning object using X-rays with at least two different energy spectrum distributions (for example, high energy spectrum and low energy spectrum) during CT scanning, hereinafter referred to as multi-spectral scanning data. For example, the above scanning data may be multi-spectral scanning data including a plurality of different energy spectral segments obtained by photon-counting CT scanning, multi-spectral scanning data obtained by performing CT scanning based on different light sources respectively, or multi-spectral scanning data obtained by performing CT scanning based on different energies respectively. The above different types of scanning data may also be different modal scanning data obtained by performing different modal scans on the same scanning object. Different modal scans include CT scanning, Positron Emission Tomography (PET) scanning, etc. For example, a PET-CT device may be used to scan a scanning object, so that the obtained different types of scanning data include PET scanning data obtained by performing PET scanning and CT scanning data obtained by performing CT scanning on the same scanning object respectively. The above different types of scanning data may also be different base material images obtained by performing different material decomposition processes on the same CT scanning data. In addition, the above different types of scanning data may also be preprocessed data obtained by performing other preprocessing on the scanning data. The scanning data may be forward projection raw data or filtered back-projection images obtained after scanning.

In some embodiments, the scanning data includes scanning data of a plurality of different energy spectra. Scanning data of each energy spectrum corresponds to an energy spectral segment, such as 30-60 kv, 60-140 kv, etc.

In step S220, processing is performed on the scanning data by using a predetermined deep learning network, to obtain a processing result of the scanning data. The deep learning network is configured to extract features from scanning data of each energy spectrum among the scanning data of a plurality of different energy spectra, i.e., perform feature extraction on each single type of scanning data among the different types of scanning data (hereinafter, this feature extraction method is referred to as "separate feature extraction"), and extract features from scanning data of all energy spectra among the scanning data of a plurality of different energy spectra, i.e., perform feature extraction on all types of scanning data among the different types of scanning data (hereinafter, this feature extraction method is referred to as "common feature extraction"). Categories of features extracted through separate feature extraction and features extracted through common feature extraction may be the same or different. These categories of features include local texture, edge, color information, etc., as well as global shape, structure, semantic information, etc. Separate feature extraction is to perform feature extraction on a single type of scanning data, and the extracted features are used only by the scanning data corresponding to energy spectrum. For example, a processing result corresponding to the scanning data of the energy spectrum is obtained using the separately extracted features. Common feature extraction is to perform feature extraction on a plurality of categories of scanning data, and the extracted features are shared by the plurality of categories of scanning data. For example, a processing result corresponding to the scanning data of the plurality of different energy spectra is obtained using the common features. In some embodiments, the processing result corresponding to the scanning data of the plurality of different energy spectra includes respective processing results corresponding to each of the scanning data of a plurality of different energy spectra.

In some embodiments, the deep learning network includes both a single extraction layer for performing separate feature extraction on a single type of scanning data and a common extraction layer for performing common feature extraction on a plurality of types of scanning data. For example, for scanning data of two types of energy spectra, such as first energy spectrum scanning data and second energy spectrum scanning data, the deep learning network may have two extraction layers for extracting features of the scanning data of two types of energy spectra. The first extraction layer extracts features that can be shared by the first energy spectrum scanning data and the second energy spectrum scanning data from the first energy spectrum scanning data and the second energy spectrum scanning data, and the second extraction layer extracts features that are used only by the first energy spectrum scanning data from the first energy spectrum scanning data, or extracts features that are used only by the second energy spectrum scanning data from the second energy spectrum scanning data. In this case, the first extraction layer may be referred to as a common extraction layer, and the second extraction layer may be referred to as a single extraction layer.

The above deep learning network may be any neural network used for medical image processing, such as a medical image segmentation model Unet++, a dense convolutional network (DenseNet), a residual network (ResNet), etc., which is not specifically limited in this embodiment. A single type of scanning data refers to one type of scanning data among the above different types of scanning data, for example, one type of scanning data corresponding to one energy spectrum among a scanning data corresponding to a plurality of energy spectra respectively. A plurality of types of scanning data refers to some or all types of scanning data among the plurality of different types of scanning data with consistent image structures.

The deep learning network includes a single extraction layer for performing separate feature extraction on scanning data of each energy spectrum among the different types of scanning data, and a common extraction layer for performing common feature extraction on the different types of scanning data. When performing separate feature extraction on scanning data of each energy spectrum among the different types of scanning data, first the scanning data are distinguished by the deep learning network according to different types, and then separate feature extraction are performed on scanning data of each energy spectrum. When performing common feature extraction on the different types of scanning data, the different types of scanning data are not distinguished by the deep learning network, but the features of the different types of scanning data are jointly extracted.

In addition, the above joint processing performed on the different types of scanning data using the predetermined deep learning network may include denoising processing on input different types of scanning data to improve the image quality of the scanning data, for example, performing segmentation or recognition of a region of interest on the input different types of scanning data to output a corresponding segmented image or recognition result, or performing scan result analysis on the input scanning data, etc. Scan result analysis includes performing structural analysis on the image of the scanning object to identify lesions, performing energy spectrum analysis on the image of the scanning object to identify material components, etc. Based on this, a structure of the above deep learning network will be different according to different application scenarios, and structures and specific implementations of the above single extraction layer and common extraction layer will also be different. For multi-spectral scanning data, by performing joint processing such as denoising, image segmentation, and scan result analysis on the multi-spectral scanning data, an accuracy of the processing results can be improved. For example, joint denoising of multi-spectral scanning data can not only enable simultaneous denoising of scanning data of a plurality of different energy spectra, improve denoising efficiency, but also improve denoising performance and improve denoising results based on the extraction of common features between data features of the scanning data of the plurality of different energy spectra and respective features of scanning data of all energy spectra. Therefore, for multi-spectral scanning data, the accuracy of processing results, processing performance, and processing efficiency can be improved based on the above method.

In the above steps S210 to S220, scanning data are acquired, and a predetermined deep learning network is used to perform joint processing on the scanning data to obtain processing results of different types of scanning data. The deep learning network used includes both a single extraction layer for performing separate feature extraction on a single type of scanning data and a common extraction layer for performing common feature extraction on a plurality of types of scanning data. As a result, by jointly inputting different types of scanning data with consistent image structures such as multi-spectral scanning data, the deep learning network can be used to simultaneously process the different types of scanning data with consistent image structures such as multi-spectral scanning data, thereby improving the accuracy of the output results of the deep learning network and the efficiency of scanning data analysis.

In an embodiment, the above step S210 of acquiring scanning data may include: acquiring initial scanning data obtained by photon-counting computed tomography; dividing the initial scanning data into a plurality of different energy spectral segments (for example, at least two energy spectral segments) to obtain scanning data corresponding to the plurality of different energy spectral segments respectively. For the division method of energy spectral segments, it can be determined based on the image quality of each energy spectral segment after image reconstruction and/or the number of photons obtained in each energy spectral segment. Specifically, after one photon-counting CT scan, obtained energy spectrum data are divided into a plurality of different energy spectral segments, for example, divided into at least two energy spectral segments. For example, on a condition that a total energy spectrum of 140 kv is divided into two energy spectral segments, the first energy spectral segment may be 30-60 kv, and the second energy spectral segment may be 60-140 kv, or the first energy spectral segment may be 20-65 kv, and the second energy spectral segment may be 65-140 kv. Each energy spectral segment corresponds to scanning data with an energy spectrum, thereby acquiring scanning data corresponding to the plurality of different energy spectral segments respectively. As a result, in this embodiment, scanning data corresponding to the plurality of different energy spectral segments can be obtained based on once photon-counting CT scan.

In another embodiment, the above step S210 of acquiring scanning data may include: acquiring scanning data corresponding to a plurality of energy spectra (for example, at least two energy spectra) respectively based on energy spectral computed tomography. For example, computed tomography is performed using at least two different energies respectively to obtain different scanning data corresponding to different energy spectra respectively, or computed tomography is performed based on two different light sources respectively to obtain different scanning data corresponding to different energy spectra respectively.

In an embodiment, before performing joint processing on the different types of scanning data by using the predetermined deep learning network, to obtain processing results of the different types of scanning data, the above method for processing scanning data may further include: directly inputting the different types of scanning data into a model of the deep learning network, or inputting the different types of scanning data into different channels of the model respectively. In some embodiments, the different types of scanning data may be combined in the image channel dimension or the image spatial dimension, and the combined scanning data are used as an input to the deep learning network. Combining in the image channel dimension means combining different types of scanning data in the image channel dimension, and combining in the image spatial dimension means combining different types of scanning data in the image spatial dimension. For example, scanning data of the same energy spectrum may be input into the same channel of the model.

In an embodiment, before performing joint processing on the different types of scanning data by using the predetermined deep learning network, to obtain processing results of the different types of scanning data, the above method for processing scanning data may further include: using the different types of scanning data as inputs to the deep learning network respectively. That is, the different types of scanning data may correspond to different inputs of the deep learning network respectively, and the different types of scanning data are input into the deep learning network simultaneously, so that the deep learning network internally processes the different scanning data.

In an embodiment, the above step S220 of performing joint processing on the different types of scanning data by using a predetermined deep learning network, to obtain processing results of the different types of scanning data may include: separately extracting data features of the scanning data of each energy spectrum by using the single extraction layer of the deep learning network, to obtain single data features corresponding to the different types of scanning data respectively, jointly extracting data features of the different types of scanning data by using the common extraction layer of the deep learning network, to obtain common data features, and obtaining processing results of the different types of scanning data based on the single data features and the common data features.

Both the single extraction layer and the common extraction layer are feature extraction layers in the deep learning network. For different deep learning networks, the specific implementation of the structure of the above feature extraction layers is different, which is not specifically limited in this embodiment. The data features extracted by the feature extraction layers refer to certain image features extracted by convolution kernels in the neural network structure, such as image information such as image edges/small blood vessels. In the single extraction layer, it is necessary to distinguish the data sources of different types of scanning data to separately extract data features of scanning data of each energy spectrum. For example, for multi-spectral scanning data, data features of scanning data of each energy spectrum are separately extracted. The single extraction layer is mainly configured to extract features from a single type of scanning data to perform network training on the single type of scanning data. Single data features refer to data features extracted from a single type of scanning data by the single extraction layer. In the common extraction layer, the scanning data are not distinguished, and data features of all scanning data are jointly extracted. The common extraction layer is mainly configured to extract common features of the scanning data of the plurality of different energy spectra (for example, the scanning data of the plurality of different energy spectra included in multi-spectral images) from different types of scanning data to perform network training on the different types of scanning data. Common data features refer to common data features extracted from the different types of scanning data by the common extraction layer.

By the deep learning network, separate optimization processing may be performed on each type of scanning data based on the above single data features. For example, in the case where there are first type scanning data and second type scanning data, optimization processing may be performed on the first type scanning data based on first single data features extracted from the first type scanning data, and optimization processing may be performed on the second type scanning data based on second single data features extracted from the second type scanning data. In addition, by the deep learning network, joint optimization processing may be performed on different types of scanning data based on the above common data features, so that the optimization results of different types of scanning data interact and influence each other. For example, in the case where there are first type scanning data and second type scanning data, the first type scanning data and the second type scanning data may be separately improved based on common data features jointly extracted from the first type scanning data and the second type scanning data (including first common data features extracted from the first type scanning data and second common data features extracted from the second type scanning data), i.e., the first type scanning data are improved based on the first common data features and the second common data features, and the second type scanning data are improved based on the first common data features and the second common data features. The above separate optimization processing and common optimization processing include, but are not limited to, the following optimization processing: improving the image quality of scanning data (including denoising, edge smoothing, etc.); improving the segmentation of a region of interest in the scanning data; and improving the recognition of a region of interest in the scanning data, etc.

Based on this, in this embodiment, since the deep neural network includes a single extraction layer and a common extraction layer, it is possible to avoid excessive fluctuations in processing results between different types of scanning data while processing different types of scanning data in parallel, which affects the consistency of the structure of the scanning object reflected by the different types of scanning data.

Hereinafter, a description will be given by taking denoising processing of multi-spectral CT scanning data as an example. After obtaining CT scanning data of two types of energy spectra, they are input into a deep learning network. By the deep learning network, single data features of the CT scanning data of two types of energy spectra are separately extracted, denoising processing is performed on each type of CT scanning data based on the single data features, common data features of the CT scanning data of two types of energy spectra are jointly extracted, and common denoising processing on the common data features is performed. As a result, by the deep learning network, it can not only realize simultaneous denoising of the CT scanning data of two types of energy spectra, but also ensure the structural consistency between the CT scanning data of two types of energy spectra and avoid excessive fluctuations in CT values.

In particular, in an embodiment, the above method for processing scanning data may further include: on a condition that the above different types of scanning data are multi-spectral data of at least two energy spectra that have not undergone material decomposition, performing material decomposition on the output result of the deep learning network, and constraining the material decomposition process based on a predetermined loss function to obtain a material map corresponding to the multi-spectral data, and on a condition that the above different types of scanning data are material decomposition data, converting the output result of the deep learning network into target energy spectral data, and calculating a loss function of the target energy spectral data using a loss function structure in the deep learning network.

The above material decomposition refers to a process of decomposing CT scanning data of a mixture into element maps according to chemical composition, such as decomposing into a water map, an iodine map, etc. The specific material decomposition to be performed depends on actual application requirements. The above multi-spectral data of at least two energy spectra are at least two different types of CT scanning data corresponding to at least two energy spectra respectively. In this embodiment, when the scanning data are CT scanning data, material decomposition is performed on the output result processed by the deep learning network, for example, after denoising the multi-spectral data using the deep learning network, material decomposition is performed on the denoised CT scanning data. Based on the above material decomposition, a more accurate and clear expression can be provided for the scan result, so as to facilitate subsequent analysis and judgment based on the scan result.

When using a deep learning network to process scanning data, it is often necessary to verify the accuracy of the output of the deep learning network with reference to a ground truth. For scanning data of two types of energy spectra, such as first energy spectrum scanning data and second energy spectrum scanning data, ground truth may be set for the scanning data of two types of energy spectra respectively, and a model based on the ground truth will output results corresponding to the first energy spectrum scanning data and results corresponding to the second energy spectrum scanning data respectively. For another example, a ground truth may be set only for the first energy spectrum scanning data, and then the model outputs only results corresponding to the first energy spectrum scanning data, or a ground truth may be set only for the second energy spectrum scanning data, and then the model outputs only results corresponding to the second energy spectrum scanning data. The ground truth can be expressed in a matrix form in mathematics, with a size consistent with the scan result. Therefore, when performing material decomposition on the output result of the deep learning network, it is also necessary to perform material decomposition on the ground truth. To improve the smoothness of image edges after material decomposition and to improve the quality of the material map, in this embodiment, a loss function is used to constrain the material decomposition process. Specifically, the loss function is a loss value between a decomposition result obtained by multiplying the output result of the deep learning network by a material decomposition matrix and a decomposition result obtained by multiplying the ground truth by the material decomposition matrix. The loss function is specifically as follows:

$$\begin{pmatrix} M_{out,1} & M_{gold,1} \\ \cdots & \cdots \\ M_{out,m} & M_{gold,m} \end{pmatrix} = \begin{pmatrix} \mu_{11} & \mu_{1n} \\ \cdots & \cdots \\ \mu_{m1} & \mu_{mn} \end{pmatrix} * \begin{pmatrix} Out_1 & Gold_1 \\ \cdots & \cdots \\ Out_n & Gold_n \end{pmatrix} \quad (1)$$

$$Loss = L(M_{out,1} - M_{gold,1}, M_{out,2} - M_{gold,2}, ..., M_{out,m} - M_{gold,m}) \quad (2)$$

where $M_{out,i}$ refers to a decomposition result of the $i$-th material corresponding to the output result of the deep learning network after material decomposition, $M_{gold,i}$ refers to a decomposition result of the $i$-th material corresponding to the ground truth after material decomposition, $\mu i,j$ refers to a decomposition coefficient matrix of the base material $i$ under the energy spectrum $j$, $Out_j$ refers to data of the $j$-th energy spectrum output by the deep learning network, and $Gold_j$ refers to data of the energy spectrum $j$ in the corresponding ground truth. The above loss function may use only material decomposition results of one set of material bases, or may use material decomposition results of a plurality of sets of material bases. The above material decomposition results form a material map, which can be used for material identification and medical diagnosis. Based on this, in this embodiment, the material decomposition process is constrained based on the loss function, which can make the edges of the processed material map smoother, improve the quality of the material map, and facilitate subsequent operations on the material map.

As described above, on a condition that the above different types of scanning data input into the deep learning network are multi-spectral data of at least two energy spectra that have not undergone material decomposition, a loss function structure of material decomposition data may be added to the deep learning network. After converting the output result of the deep learning network (i.e., target energy spectral data) into material decomposition data, the loss function of the material decomposition data is calculated using the loss function structure of the material decomposition data. By adding a loss function structure of a data type (for example, material decomposition data) different from the input data type (for example, energy spectral data) to the deep learning network, the model and parameters used in the deep learning network can be further improved using loss functions of different data types, thereby making the edges of the processed material map smoother to improve the quality of the material map.

In addition, on a condition that the above different types of scanning data input into the deep learning network are material decomposition data that have undergone material decomposition, i.e., a material map, a loss function structure of target energy spectral data may be added to the deep learning network. After converting the output result of the deep learning network (i.e., a target material map) into target energy spectral data, the loss function of the target energy spectral data is calculated using the loss function structure of the target energy spectral data. As a result, the edges of the processed material map can also be made smoother to improve the quality of the material map.

In an embodiment, the joint processing performed by the deep learning network on the different types of scanning data includes at least one of the following: denoising processing on the different types of scanning data, extraction of a region of interest from the different types of scanning data, or scan result analysis on the different types of scanning data. For example, the scanning data input into the deep neural network is a noise image, and the output may be a denoised image. The input into the deep neural network is a scan image containing a region of interest, and the output is a segmented region of interest or a scan result analysis for medical analysis of the scan image.

Hereinafter, the method for processing scanning data provided by the present disclosure will be further described and explained through optional implementations.

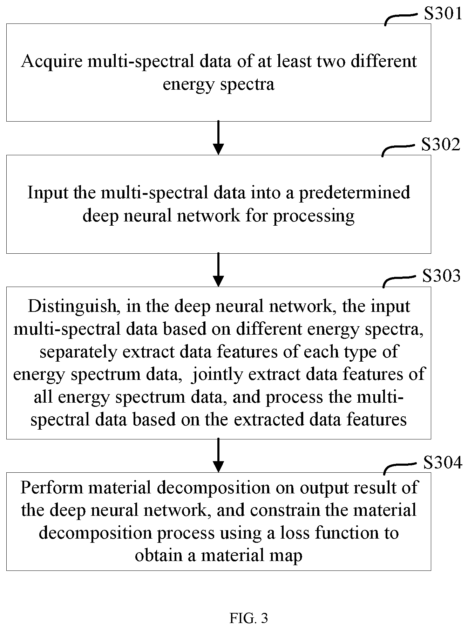

FIG. 3 is a flow diagram of a method for processing scanning data according to an embodiment of the present disclosure. As shown in FIG. 3, the method for processing scanning data includes steps S301 to S304.

In step S301, multi-spectral data of at least two different energy spectra are obtained. The energy spectrum data obtained by photon-counting CT may be divided into a plurality of different energy spectral segments to obtain multi-spectral data, or multi-spectral data may be obtained by performing energy spectral CT scanning based on different light sources or different energies respectively. The multi-spectral data may be forward projection raw data or filtered back-projection images.

In step S302, the multi-spectral data are input into a predetermined deep neural network for processing.

In step S303, in the deep neural network, the input multi-spectral data are distinguished based on different energy spectra, data features of each type of energy spectrum data are separately extracted, data features of all energy spectrum data are jointly extracted, and the multi-spectral data are processed based on the extracted data features. Specific processing may include denoising, region of interest segmentation, scan result analysis, etc.;

In step S304, material decomposition is performed on the output result of the deep neural network, and the material decomposition process is constrained using a loss function to obtain a material map.

In the above steps S301 to S304, by performing joint processing such as denoising, image segmentation, or scan result analysis on the multi-spectral data, processing performance can be improved while improving processing efficiency. In addition, a material map is obtained by constraining the material decomposition process based on the loss function, which can make the edges of the processed material map smoother to improve the quality of the material map.

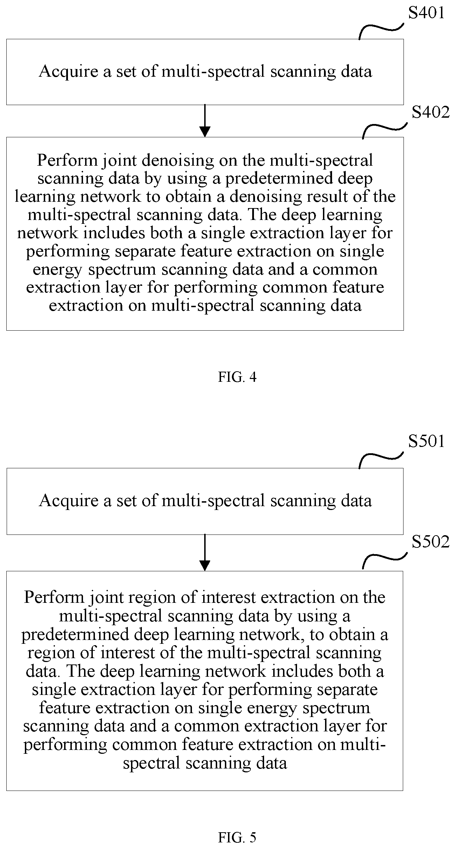

In another embodiment, a method for denoising multi-spectral scanning data is provided. As shown in FIG. 4, the above denoising method may specifically include steps S401 and S402.

In step S401, a set of multi-spectral scanning data are obtained. The multi-spectral scanning data may be at least two types of multi-spectral scanning data obtained by scanning a scanning object using at least two different energy spectra during CT scanning. For example, multi-spectral scanning data including a plurality of different energy spectral segments obtained by photon-counting CT scanning, multi-spectral scanning data obtained by performing CT scanning based on different light sources respectively, or multi-spectral scanning data obtained by performing CT scanning based on different energies respectively.

In step S402, joint denoising is performed on the above multi-spectral scanning data by using a predetermined deep learning network to obtain a denoising result of the above multi-spectral scanning data. The above deep learning network includes both a single extraction layer for performing separate feature extraction on single energy spectrum scanning data and a common extraction layer for performing common feature extraction on multi-spectral scanning data. The above deep learning network may be any neural network used for medical image denoising, such as a Convolutional Neural Network (CNN), a Deep Convolutional Neural Network (DCNN), etc., which is not specifically limited in this embodiment.

In the above steps S401 to S402, denoising processing can be performed on multi-spectral scanning data simultaneously, and denoising performance can be further improved based on common features of the multi-spectral scanning data while improving denoising efficiency, thereby improving denoising effect.

In another embodiment, a method for image segmentation of multi-spectral scanning data is provided. As shown in FIG. 5, the above image segmentation method may specifically include steps S501 and S502.

In step S501, a set of multi-spectral scanning data are acquired. The multi-spectral scanning data may be different types of multi-spectral scanning data obtained by scanning a scanning object using at least two different energy spectra during CT scanning.

In step S502, joint region of interest extraction is performed on the above multi-spectral scanning data by using a predetermined deep learning network, to obtain a region of interest of the above multi-spectral scanning data. The above deep learning network includes both a single extraction layer for performing separate feature extraction on single energy spectrum scanning data and a common extraction layer for performing common feature extraction on multi-spectral scanning data. The above deep learning network includes both a single extraction layer for performing separate feature extraction on single energy spectrum scanning data and a common extraction layer for performing common feature extraction on multi-spectral scanning data. The above deep learning network may be any neural network used for medical image segmentation, such as a medical image segmentation model Unet, Unet++, Vnet, etc., which is not specifically limited in this embodiment.

It should be understood that although the various steps in the flow diagrams involved in the above embodiments are displayed in sequence according to the arrows, these steps are not necessarily executed in sequence according to the arrow instructions. Unless explicitly stated in this document, there is no strict order restriction on the execution of these steps, and these steps can be executed in other orders. Moreover, at least part of the steps in the flow diagrams involved in the above embodiments may include multiple steps or multiple stages. These steps or stages are not necessarily executed at the same time, but can be executed at different times. The execution order of these steps or stages is not necessarily sequential, but can be rotated or alternately executed with at least part of the steps or stages in other steps or other steps.

In another aspect of the present disclosure, a device for processing scanning data is further provided. The implementation for solving problems provided by the device is similar to the implementation disclosed in the above method, so specific limitations in one or more device embodiments provided below can be referred to the limitations on the method for processing scanning data in the above, which will not be repeated here. The terms "module", "unit", "sub-unit", etc. used below can realize a combination of software and/or hardware with predetermined functions. Although the apparatus described in the following embodiments is preferably implemented by software, implementation by hardware or a combination of software and hardware is also possible and conceived.

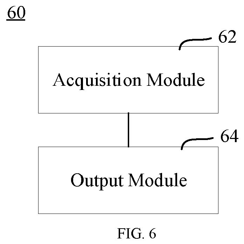

FIG. 6 is a block diagram of a configuration of a device 60 for processing scanning data according to an embodiment of the present disclosure. As shown in FIG. 6, the device 60 for processing scanning data includes: an acquisition module 62 and an output module 64. The acquisition module 62 is used to acquire scanning data. The output module 64 is used to perform processing on the scanning data by using a predetermined deep learning network, to obtain a processing result of the scanning data. The scanning data includes scanning data of a plurality of different energy spectra. The deep learning network is configured to extract features from scanning data of each energy spectrum among the scanning data of a plurality of different energy spectra, and extract features from scanning data of all energy spectra among the scanning data of a plurality of different energy spectra. Specifically, the deep learning network includes both a single extraction layer for extracting features from scanning data of each energy spectrum and a common extraction layer for extracting features from scanning data of a plurality of different energy spectra. Features extracted from scanning data of each energy spectrum by the single extraction layer are used only by the scanning data of each energy spectrum. Features extracted from the scanning data of the plurality of different energy spectra by the common extraction layer are shared by the scanning data of a plurality of different energy spectra.

In some embodiments, the acquisition module 62 is further configured to acquire initial scanning data obtained by photon-counting computed tomography, and divide the initial scanning data into a plurality of different energy spectral segments to acquire the scanning data of the plurality of different energy spectra corresponding to the plurality of different energy spectral segments respectively.

In some embodiments, the acquisition module 62 is further configured to acquire the scanning data of the plurality of different energy spectra corresponding to a plurality of energy spectra respectively based on energy spectral computed tomography.

In some embodiments, the acquisition module 62 is further configured to perform computed tomography by using at least two different energies respectively to obtain scanning data corresponding to different energy spectra respectively, or perform computed tomography based on two different light sources respectively to obtain scanning data corresponding to different energy spectra respectively.

In some embodiments, the acquisition module 62 is further configured to use the scanning data of the plurality of different energy spectra as inputs to the deep learning network respectively.

In some embodiments, the output module 64 is further configured to separately extract data features of the scanning data of each energy spectrum by using the single extraction layer of the deep learning network, to obtain single data features corresponding to the scanning data of the plurality of different energy spectra respectively; jointly extract data features of different types of scanning data by using the common extraction layer of the deep learning network, to obtain common data features, and obtain processing results of the scanning data of the plurality of different energy spectra based on the single data features and the common data features.

In some embodiments, the output module 64 is further configured to perform separate optimization processing on the scanning data of each energy spectrum by using the deep learning network based on the single data features, and perform joint optimization processing on the scanning data of the plurality of different energy spectra by using the deep learning network based on the common data features.

In some embodiments, the output module 64 is further configured to perform, on a condition that the scanning data are multi-spectral data that have not undergone material decomposition, material decomposition on output result of the deep learning network, and constrain a material decomposition process based on a predetermined loss function to obtain a material map corresponding to the multi-spectral data, or convert, on a condition that the scanning data are material decomposition data, the output result of the deep learning network into target energy spectral data, and calculate a loss function of the target energy spectral data using a loss function structure in the deep learning network.

In some embodiments, the output module 64 is further configured to perform at least one of the following processes: denoising processing on the scanning data of a plurality of different energy spectra, extraction of a region of interest from the scanning data of a plurality of different energy spectra, or scan result analysis on the scanning data of a plurality of different energy spectra.

It should be noted that the above modules may be functional modules or program modules, and may be implemented by software or hardware. For modules implemented by hardware, the above modules may be located in the same processor; or the above modules may be located in different processors according to any combination.

It should be noted that specific examples in this embodiment can refer to the examples described in the above embodiments and optional implementations, which will not be repeated in this embodiment.

In a further aspect of the present disclosure, a computer apparatus is further provided, which may be the terminal shown in FIG. 1. The memory 104 of the terminal stores a computer program for processing scanning data. When the processor executes the computer program, the method for processing scanning data according to any one of the above embodiments is implemented.

In an exemplary embodiment, when the processor executes the computer program, the following is implemented: acquiring scanning data, and performing joint processing on the scanning data by using a predetermined deep learning network, to obtain a processing result of the scanning data. The scanning data includes scanning data of a plurality of different energy spectra. The deep learning network is configured to extract features from scanning data of each energy spectrum among the scanning data of the plurality of different energy spectra, and extract features from scanning data of all energy spectra among the scanning data of the plurality of different energy spectra.

In some embodiments, the deep learning network includes both a single extraction layer for extracting features from the scanning data of each energy spectrum and a common extraction layer for extracting features from the scanning data of all energy spectra.

In some embodiments, features extracted from the scanning data of each energy spectrum are used only by the scanning data corresponding to energy spectrum, and features extracted from the scanning data of all energy spectra are shared by the scanning data of the plurality of different energy spectra.

In some embodiments, acquiring the scanning data includes: acquiring initial scanning data obtained by photon-counting computed tomography, and dividing the initial scanning data into a plurality of different energy spectral segments to acquire the scanning data of the plurality of different energy spectra corresponding to the plurality of different energy spectral segments respectively.

In some embodiments, acquiring the scanning data includes acquiring the scanning data of the plurality of different energy spectra corresponding to a plurality of energy spectra respectively based on energy spectral computed tomography.

In some embodiments, acquiring the scanning data of the plurality of different energy spectra corresponding to the plurality of energy spectra respectively based on energy spectral computed tomography includes performing computed tomography using at least two different energies respectively to obtain scanning data corresponding to different energy spectra respectively, or performing computed tomography based on two different light sources respectively to obtain scanning data corresponding to different energy spectra respectively.

In some embodiments, before performing processing on the scanning data by using the predetermined deep learning network, to obtain the processing result of the scanning data, the method further includes using the scanning data as inputs to the deep learning network respectively.

In some embodiments, performing processing on the scanning data by using the predetermined deep learning network, to obtain the processing result of the scanning data includes: separately extracting data features of the scanning data of each energy spectrum by using the single extraction layer of the deep learning network, to obtain single data features corresponding to the scanning data of the plurality of different energy spectra respectively, jointly extracting data features of the scanning data of the plurality of different energy spectra by using the common extraction layer of the deep learning network, to obtain common data features, and obtaining processing results of the scanning data of the plurality of different energy spectra based on the single data features and the common data features.

In some embodiments, performing joint processing on the scanning data by using a predetermined deep learning network, to obtain the processing result of the scanning data further includes: performing separate optimization processing on scanning data of each energy spectrum by using the deep learning network based on the single data features, and performing joint optimization processing on the scanning data of the plurality of different energy spectra by using the deep learning network based on the common data features.

In some embodiments, when the processor executes the computer program, the following is further implemented: on a condition that the scanning data are multi-spectral data that have not undergone material decomposition, performing material decomposition on output result of the deep learning network, and constraining a process of the material decomposition based on a predetermined loss function to obtain a material map corresponding to the multi-spectral data, or on a condition that the scanning data are material decomposition data, converting the output result of the deep learning network into target energy spectral data, and calculating a loss function of the target energy spectral data using a loss function structure in the deep learning network.

In some embodiments, when the processor executes the computer program, at least one of the following processes is further implemented: denoising processing on the scanning data of the plurality of different energy spectra, extraction of a region of interest from the scanning data of the plurality of different energy spectra, or scan result analysis on the scanning data of the plurality of different energy spectra.

In a further aspect of the present disclosure, a computer-readable storage medium is further provided. A computer program is stored on the storage medium. When the computer program is executed by a processor, the processor is caused to implement the method for processing scanning data according to any one of the above embodiments.

Those of ordinary skill in the art can understand that all or part of the processes in the methods of the above embodiments can be implemented by instructing relevant hardware through a computer program. The computer program can be stored in a non-transitory computer-readable storage medium. When the computer program is executed, it may include the processes of the embodiments of the above methods. Any reference to the memory, database, or other medium used in the various embodiments provided in the present disclosure may include at least one of non-transitory memory or transitory memory. Non-transitory memory may include read-only memory (ROM), magnetic tape, floppy disk, flash memory, optical memory, high-density embedded non-transitory memory, resistive random access memory (ReRAM), magnetoresistive random access memory (MRAM), ferroelectric random access memory (FRAM), phase change memory (PCM), graphene memory, etc. Transitory memory may include random access memory (RAM) or external cache memory, etc. By way of illustration and not limitation, RAM may be in various forms, such as static random access memory (SRAM) or dynamic random access memory (DRAM), etc. The databases involved in the various embodiments provided in the present disclosure may include at least one of a relational database or a non-relational database. Non-relational databases may include databases based on blockchains, etc., without limitation. The processors involved in the various embodiments provided in the present disclosure may be general-purpose processors, central processing units, graphics processors, digital signal processors, programmable logic devices, data processing logic devices based on quantum computing, artificial intelligence (AI) processors, etc., without limitation.

The above-described embodiments only express several implementation modes of the present disclosure, and their descriptions are relatively specific and detailed, but they should not be understood as limiting the scope of the present disclosure. It should be noted that for those of ordinary skill in the art, without departing from the concept of the present disclosure, several modifications and improvements can also be made, which all fall within the protection scope of the present disclosure. Therefore, the protection scope of the present disclosure should be subject to the appended claims.

**Claims**

1. A method for processing scanning data, the method comprising:

   acquiring scanning data; and
   performing processing on the scanning data by using a predetermined deep learning network, to obtain a processing result of the scanning data,
   wherein the scanning data comprises scanning data of a plurality of different energy spectra, and
   wherein the deep learning network is configured to extract features from scanning data of each energy spectrum among the scanning data of the plurality of different energy spectra, and extract features from scanning data of all energy spectra among the scanning data of the plurality of different energy spectra.

2. The method for processing scanning data according to claim 1, wherein the deep learning network comprises both a single extraction layer for extracting features from the scanning data of each energy spectrum and a common extraction layer for extracting features from the scanning data of all energy spectra.

3. The method for processing scanning data according to claim 1, wherein features extracted from the scanning data of each energy spectrum are used only by the scanning data corresponding to energy spectrum, and features extracted from the scanning data of all energy spectra are shared by the scanning data of the plurality of different energy spectra.

4. The method for processing scanning data according to claim 1, wherein the scanning data are obtained by a medical scanning device, and the medical scanning device comprises at least one of a photon-counting computed tomography device, an energy spectral computed tomography device, a direct digital radiography device, a single-photon emission computed tomography device, or a positron emission tomography device.

5. The method for processing scanning data according to claim 1, wherein acquiring the scanning data comprises:

acquiring initial scanning data obtained by photon-counting computed tomography; and
dividing the initial scanning data into a plurality of different energy spectral segments to acquire the scanning data of the plurality of different energy spectra corresponding to the plurality of different energy spectral segments respectively.

6. The method for processing scanning data according to claim 1, wherein acquiring the scanning data comprises:
acquiring the scanning data of the plurality of different energy spectra corresponding to a plurality of energy spectra respectively based on energy spectral computed tomography.

7. The method for processing scanning data according to claim 6, wherein acquiring the scanning data of the plurality of different energy spectra corresponding to the plurality of energy spectra respectively based on energy spectral computed tomography comprises:

performing computed tomography using at least two different energies respectively to obtain scanning data corresponding to different energy spectra respectively; or
performing computed tomography based on two different light sources respectively to obtain scanning data corresponding to different energy spectra respectively.

8. The method for processing scanning data according to any one of claims 1 to 7, wherein before performing processing on the scanning data by using the predetermined deep learning network, to obtain the processing result of the scanning data, the method further comprises:
using the scanning data as inputs to the deep learning network.

9. The method for processing scanning data according to any one of claims 2 to 8, wherein performing processing on the scanning data by using the predetermined deep learning network, to obtain the processing result of the scanning data comprises:

separately extracting data features of the scanning data of each energy spectrum by using the single extraction layer of the deep learning network, to obtain single data features corresponding to the scanning data of the plurality of different energy spectra respectively;
jointly extracting data features of the scanning data of the plurality of different energy spectra by using the common extraction layer of the deep learning network, to obtain common data features; and
obtaining processing results of the scanning data of the plurality of different energy spectra based on the single data features and the common data features.

10. The method for processing scanning data according to claim 9, wherein performing processing on the scanning data by using the predetermined deep learning network, to obtain the processing result of the scanning data further comprises:

performing separate optimization processing on scanning data of each energy spectrum by using the deep learning network based on the single data features; and
performing joint optimization processing on the scanning data of the plurality of different energy spectra by using the deep learning network based on the common data features.

11. The method for processing scanning data according to any one of claims 1 to 10, wherein the method further comprises:

performing, on a condition that the scanning data are multi-spectral data that have not undergone material decomposition, material decomposition on output result of the deep learning network, and constraining a process of the material decomposition based on a predetermined loss function to obtain a material map corresponding to the multi-spectral data; or

converting, on a condition that the scanning data are material decomposition data, the output result of the deep learning network into target energy spectral data, and calculating a loss function of the target energy spectral data using a loss function structure in the deep learning network.

12. The method for processing scanning data according to any one of claims 1 to 11, wherein the processing performed by the deep learning network on the scanning data comprises at least one of the following processes:

denoising processing on the scanning data of the plurality of different energy spectra;
extraction of a region of interest from the scanning data of the plurality of different energy spectra; or
scan result analysis on the scanning data of the plurality of different energy spectra.

13. A device for processing scanning data, comprising:

an acquisition module configured to acquire scanning data; and
an output module configured to perform processing on the scanning data by using a predetermined deep learning network, to obtain a processing result of the scanning data,
wherein the scanning data comprises scanning data of a plurality of different energy spectra, and
wherein the deep learning network is configured to extract features from scanning data of each energy spectrum among the scanning data of the plurality of different energy spectra, and extract features from scanning data of all energy spectra among the scanning data of the plurality of different energy spectra.

14. The device for processing scanning data according to claim 13, wherein the deep learning network comprises both a single extraction layer for extracting features from the scanning data of each energy spectrum and a common extraction layer for extracting features from the scanning data of all energy spectra.

15. The device for processing scanning data according to claim 14, wherein features extracted from the scanning data of each energy spectrum are used only by the scanning data corresponding to energy spectrum, and features extracted from the scanning data of all energy spectra are shared by the scanning data of the plurality of different energy spectra.

16. The device for processing scanning data according to claim 13, wherein the acquisition module is further configured to:

acquire initial scanning data obtained by photon-counting computed tomography; and
divide the initial scanning data into a plurality of different energy spectral segments to acquire the scanning data of the plurality of different energy spectra corresponding to the plurality of different energy spectral segments respectively.

17. The device for processing scanning data according to claim 13, wherein the acquisition module is further configured to:
acquire the scanning data of the plurality of different energy spectra corresponding to a plurality of energy spectra respectively based on energy spectral computed tomography.

18. The device for processing scanning data according to claim 17, wherein the acquisition module is further configured to:

perform computed tomography using at least two different energies respectively to obtain scanning data corresponding to different energy spectra respectively; or
perform computed tomography based on two different light sources respectively to obtain scanning data corresponding to different energy spectra respectively.

19. The device for processing scanning data according to any one of claims 13 to 18, wherein the acquisition module is further configured to:
use the scanning data of the plurality of different energy spectra as inputs to the deep learning network respectively.

20. The device for processing scanning data according to any one of claims 13 to 19, wherein the output module is further configured to:

separately extract data features of the scanning data of each energy spectrum by using the single extraction layer of the deep learning network, to obtain single data features corresponding to the scanning data of the plurality of different energy spectra respectively;
jointly extract data features of different types of scanning data by using the common extraction layer of the deep learning network, to obtain common data features; and
obtain processing results of the scanning data of the plurality of different energy spectra based on the single data features and the common data features.

21. The device for processing scanning data according to claim 20, wherein the output module is further configured to:

perform separate optimization processing on the scanning data of each energy spectrum by using the deep learning network based on the single data features; and
perform joint optimization processing on the scanning data of the plurality of different energy spectra by using the deep learning network based on the common data features.

22. The device for processing scanning data according to any one of claims 13 to 21, wherein the output module is further configured to:

perform, on a condition that the scanning data are multi-spectral data that have not undergone material decomposition, material decomposition on output result of the deep learning network, and constrain a material decomposition process based on a predetermined loss function to obtain a material map corresponding to the multi-spectral data; or
convert, on a condition that the scanning data are material decomposition data, the output result of the deep learning network into target energy spectral data, and calculate a loss function of the target energy spectral data using a loss function structure in the deep learning network.

23. The device for processing scanning data according to any one of claims 13 to 22, wherein the output module is further configured to perform at least one of the following:

denoising processing on the scanning data of a plurality of different energy spectra;
extraction of a region of interest from the scanning data of a plurality of different energy spectra; or
scan result analysis on the scanning data of a plurality of different energy spectra.

24. A computer apparatus, comprising a processor, and a memory storing a computer program executable by the processor, wherein the processor, when executing the computer program, implements the method for processing scanning data according to any one of claims 1 to 12.

25. A computer-readable storage medium having a computer program stored thereon, wherein the computer program, when executed by a processor, causes the processor to implement the method for processing scanning data according to any one of claims 1 to 12.

FIG. 1

FIG. 2

```
                                                    ┌─ S301
┌────────────────────────────────────────────────┐
│   Acquire multi-spectral data of at least two different
│                  energy spectra                  │
└────────────────────────────────────────────────┘
                         │
                         ▼
                                                    ┌─ S302
┌────────────────────────────────────────────────┐
│   Input the multi-spectral data into a predetermined
│           deep neural network for processing      │
└────────────────────────────────────────────────┘
                         │
                         ▼
                                                    ┌─ S303
┌────────────────────────────────────────────────┐
│   Distinguish, in the deep neural network, the input
│   multi-spectral data based on different energy spectra,
│       separately extract data features of each type of
│    energy spectrum data,  jointly extract data features of
│      all energy spectrum data, and process the multi-
│       spectral data based on the extracted data features │
└────────────────────────────────────────────────┘
                         │
                         ▼
                                                    ┌─ S304
┌────────────────────────────────────────────────┐
│   Perform material decomposition on output result of
│    the deep neural network, and constrain the material
│        decomposition process using a loss function to
│                 obtain a material map             │
└────────────────────────────────────────────────┘
```

FIG. 3

S401

Acquire a set of multi-spectral scanning data

S402

Perform joint denoising on the multi-spectral scanning data by using a predetermined deep learning network to obtain a denoising result of the multi-spectral scanning data. The deep learning network includes both a single extraction layer for performing separate feature extraction on single energy spectrum scanning data and a common extraction layer for performing common feature extraction on multi-spectral scanning data

FIG. 4

S501

Acquire a set of multi-spectral scanning data

S502

Perform joint region of interest extraction on the multi-spectral scanning data by using a predetermined deep learning network, to obtain a region of interest of the multi-spectral scanning data. The deep learning network includes both a single extraction layer for performing separate feature extraction on single energy spectrum scanning data and a common extraction layer for performing common feature extraction on multi-spectral scanning data

FIG. 5

60

62

Acquisition Module

64

Output Module

FIG. 6

## INTERNATIONAL SEARCH REPORT

| | International application No. |
|---|---|
| | **PCT/CN2024/122024** |

**A. CLASSIFICATION OF SUBJECT MATTER**

G06V 10/25(2022.01)i

According to International Patent Classification (IPC) or to both national classification and IPC

**B. FIELDS SEARCHED**

Minimum documentation searched (classification system followed by classification symbols)

IPC: G06V

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 扫描, 深度学习, 残差, 卷积, 能谱, 多能谱, 高能谱, 低能谱, 高低能谱, 特征, 提取, 单一, 共同, 层, 单独, 融合, CT, scan, deep learning, residual, convolution, spectrum, multi-energy spectrum, high energy spectrum, low energy spectrum, feature, extraction, single, layer, fusion

**C. DOCUMENTS CONSIDERED TO BE RELEVANT**

| Category* | Citation of document, with indication, where appropriate, of the relevant passages | Relevant to claim No. |
|---|---|---|
| PX | CN 117333658 A (SHANGHAI UNITED IMAGING HEALTHCARE CO., LTD.) 02 January 2024 (2024-01-02)<br>description, paragraphs [0008]-[0032] | 1-25 |
| X | CN 112287970 A (SHANDONG NORMAL UNIVERSITY) 29 January 2021 (2021-01-29)<br>description, paragraphs [0006]-[0074] | 1-25 |
| A | CN 115736964 A (WUHAN UNITED IMAGING LIFE SCIENCE INSTRUMENT CO., LTD.) 07 March 2023 (2023-03-07)<br>entire document | 1-25 |
| A | CN 110675467 A (SHENZHEN INSTITUTES OF ADVANCED TECHNOLOGY, CHINESE ACADEMY OF SCIENCES) 10 January 2020 (2020-01-10)<br>entire document | 1-25 |
| A | US 2015071400 A1 (SIEMENS AKTIENGESELLSCHAFT) 12 March 2015 (2015-03-12)<br>entire document | 1-25 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/122024**

| Patent document cited in search report | | | Publication date (day/month/year) | Patent family member(s) | | | Publication date (day/month/year) |
|---|---|---|---|---|---|---|---|
| CN | 117333658 | A | 02 January 2024 | None | | | |
| CN | 112287970 | A | 29 January 2021 | None | | | |
| CN | 115736964 | A | 07 March 2023 | None | | | |
| CN | 110675467 | A | 10 January 2020 | None | | | |
| US | 2015071400 | A1 | 12 March 2015 | DE | 102013217852 | B3 | 30 October 2014 |
| | | | | CN | 104414675 | A | 18 March 2015 |

Form PCT/ISA/210 (patent family annex) (July 2022)

**EP 4 760 647 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311269305